# EUROPEAN PATENT SPECIFICATION

(11) **EP 3 849 470 B1**
(45) Date of publication and mention of the grant of the patent: **02.12.2026**
(21) Application number: 19859290.9
(22) Date of filing: 12.09.2019
(51) Int. Cl.: A61F 2/16, G02B 5/18

(54) **MULTIFOCAL INTRAOCULAR LENS**
MULTIFOKALE INTRAOKULARLINSE
LENTILLE INTRAOCULAIRE MULTIFOCALE

(30) Priority: 13.09.2018 US 201862730769 P
(43) Date of publication of application: 21.07.2021
(60) Divisional application: 23172225.7 / 4 235 233; 23180490.7 / 4 242 707; 23180491.5 / 4 254 024; 23182032.5 / 4 258 026
(73) Proprietor: Hanita Lenses Ltd., 2288500 Hanita (IL)
(72) Inventor: ATTIA, Mickael, 7655521 Rehovot (IL); BRODSKY, Alexander, 4942108 Petah Tikva (IL); KAPLAN, Natan, 7147131 Lod (IL); GROSSINGER, Israel, 9979700 Karmei Yossef (IL)
(74) Representative: Amirsehhi, Ramin
(86) International application number: PCT/IL2019/051025
(87) International publication number: WO 2020/053864

(56) References cited:
- WO-A1-2012/078763
- WO-A2-2009/027438
- US-A1- 2006 098 163
- US-A1- 2009 187 242
- US-A1- 2009 240 328
- US-A1- 2011 270 390
- US-A1- 2012 283 825
- US-B1- 6 536 899
- LENKOVA G A: "Features of optical surfaces of multifocal diffractive-refractive eye lenses", OPTOELECTRONICS, INSTRUMENTATION AND DATA PROCESSING, PLEIADES PUBLISHING, MOSCOW, vol. 53, no. 5, 16 December 2017 (2017-12-16), pages 431 - 441, XP036629198, ISSN: 8756-6990, [retrieved on 20171216], DOI: 10.3103/S8756699017050028
- GORI F ET AL: "Analytical derivation of the optimum triplicator", OPTICS COMMUNICATIONS, ELSEVIER, AMSTERDAM, NL, vol. 157, no. 1-6, 1 December 1998 (1998-12-01), pages 13 - 16, XP004150691, ISSN: 0030-4018, DOI: 10.1016/S0030-4018(98)00518-5

## Description

### CROSS REFERENCE TO RELATED APPLICATIONS

This application claims the benefit of priority to U.S. Provisional Patent Application No. 62/730,769 filed September 13, 2018, entitled "MULTIFOCAL INTRAOCULAR LENS".

### FIELD OF THE INVENTION

The present invention relates to multi-focal, diffractive, intraocular ophthalmic lenses.

### BACKGROUND

An intraocular lens (IOL) is a lens implanted in the eye as part of a treatment for cataracts or myopia. The multifocal intraocular lenses (IOLs) available are often able to restore visual function and allow eyeglasses independence after their implantation with great levels of patient satisfaction.

Multifocal IOLs correct far-vision, near-sightedness and all mid-range distances. Multifocal IOLs improve near vision by dividing light in different focuses, which changes the vision physiology because of the light dispersion that happens when it enters into the eye. Modern multifocal IOLs provide a more physiological division of light and, in this way, optimize the eyeglasses independence and also provide better vision quality and less visual symptoms for the patient. A multifocal IOL of the prior art is disclosed in US2007182921A1.

The foregoing examples of the related art and limitations related therewith are intended to be illustrative and not exclusive. Other limitations of the related art will become apparent to those of skill in the art upon a reading of the specification and a study of the figures.

### SUMMARY

The following embodiments and aspects thereof are described and illustrated in conjunction with systems, tools and methods which are meant to be exemplary and illustrative, not limiting in scope.

The invention is defined in claim 1. Preferred embodiments are defined in the dependent claims. In some embodiments, the diffractive surface includes diffractive steps designed to maintain the diffractive profile unchanged in between the steps, and wherein the diffractive steps are partially inside and partially outside the base curvature of the IOL.
In some embodiments, the diffractive, concentric rings include a repetitive pattern of diffractive profiles. In some embodiments, the profiles are asymmetrical.

In some embodiments, the diffractive rings include a repetitive pattern of a single diffractive profile. In some embodiments, the diffractive rings include a repetitive pattern of two different diffractive profiles, and wherein the diffractive rings include a single transition from a first zone having a repetitive pattern of one diffractive profile to a second peripheral zone having a repetitive pattern of a second diffractive profile.

In some embodiments, height of the diffractive surface topography of the second peripheral zone is maintained constant when advancing radially outwards in respect to the center height of the IOL. In some embodiments, the radial phase profile cross-section has an asymmetrical double-peaked geometry.

In some embodiments, thickness of the IOL is variable and the curvature is maintained. In some embodiments, thickness of the IOL is variable and the curvature is variable.

In addition to the exemplary aspects and embodiments described above, further aspects and embodiments will become apparent by reference to the figures and by study of the following detailed description.

### BRIEF DESCRIPTION OF THE FIGURES

The patent or application file contains at least one drawing executed in color. Copies of this patent or patent application publication with color drawing(s) will be provided by the Office upon request and payment of the necessary fee.

Exemplary embodiments are illustrated in referenced figures. Dimensions of components and features shown in the figures are generally chosen for convenience and clarity of presentation and are not necessarily shown to scale. The figures are listed below.
Fig. 1A is a plan view simplified illustration of a multifocal IOL; and
Fig. 1B is a cross-section view simplified illustration of a diffractive surface of a multifocal IOL according to some embodiments of the present invention;
Fig. 2A is a radial, cross-section, simplified illustration of a diffractive surface topography of a portion of the IOL; and
Fig. 2B is a graph of distribution of energy flux (power) levels over specific diffractive orders associated with the diffractive surface topography shown in Fig. 2A;
Fig. 3A is a radial, cross-section, simplified illustration of a diffractive surface topography of a portion of the IOL; and
Fig. 3B is a graph of distribution of energy flux (power) levels over specific diffractive orders associated with the diffractive surface topography shown in Fig. 3A;
Fig. 4 is a graph superimposing two radial phase profiles in accordance with some examples of the invention;
Fig. 5A is a radial, cross-section, simplified illustration of a diffractive surface topography of a portion of the IOL;
Fig. 5B is a graph of distribution of energy flux (power) levels over specific diffractive orders associated with the diffractive surface topography shown in Fig. 5A;
Fig. 6 is a graph of a simulation of a through-focus Modulation Transfer Function (MTF) of the IOL in accordance with some embodiments of the invention; and
Fig. 7 is a simplified graph illustration depicting a profile of a diffractive portion of an exemplary lens in accordance with some embodiments of the current invention.

### DETAILED DESCRIPTION

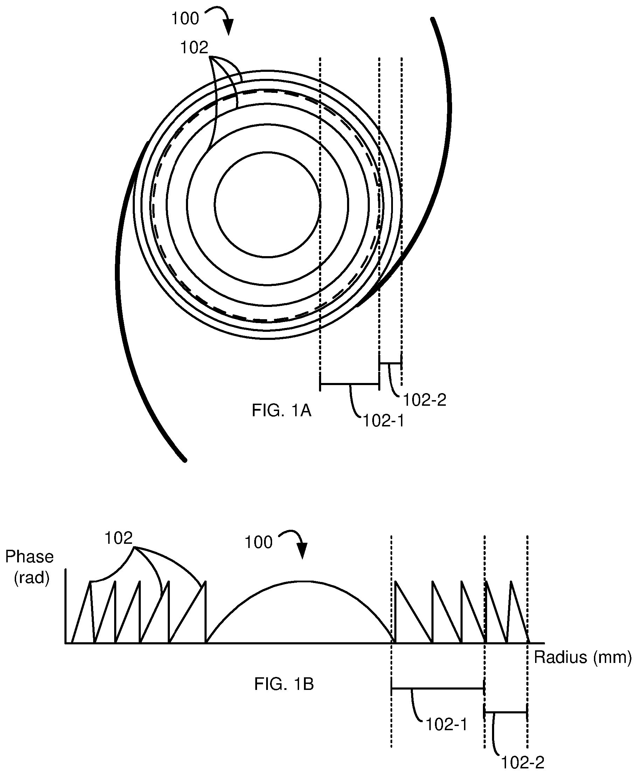

For purposes of better understanding the present invention, as illustrated in Figures 1A to 6 of the drawings, reference is first made to the construction and operation of a multifocal intraocular lens (IOL).

Multifocal Intraocular lenses (IOL) are designed such that to produce one, two, three or more diffractive orders or foci on the optical axis such that each image focal point is formed on the retina when an object associated with the image is located at a corresponding distance from the eye. Most of the intraocular lenses are designed to have a refractive (lens) focal point and energy flux (power) that corresponds to far vision i.e. the spherical/aspherical surfaces of the lens are designed to focus an image of an object on the retina when the object is located at the far-sighted distance from the eye (>5-6 meters). The diffractive surfaces in most bifocal and trifocal lenses create additional foci at the near (30-40cm) and intermediate distances (60-80cm).

Most refractive-diffractive IOLs have the same general structure - a lens comprising multiple rings, each ring having a diffractive profile that is scaled to the ring width. The diffractive profiles are repeated along the radius for each Fresnel zone. In some cases, the rings are also scaled vertically (apodization).

According to an aspect of the invention there is provided an IOL comprising diffractive steps which are partially inside and partially outside the base curvature of the IOL. In some embodiments, the thickness of the IOL is variable and the curvature is constant. In some embodiments, change in curvature is produced by optimization of the sphericity between steps.

According to an aspect of some embodiments of the present invention there is provided an IOL that has a spherical surface and an aspherical surface. According to the claimed subject-matter, the IOL comprises five diffractive orders on the optical axis.

In some embodiments, the IOL comprises a diffractive pattern on one or more surfaces of the lens. In some embodiments, the diffractive pattern comprises a repetitive pattern of diffractive profiles. In some embodiments, the profiles are asymmetrical.

Reference is now made to Figs. 1A and 1B (not drawn to scale), in which Fig. 1A is a plan view simplified illustration of a multifocal IOL and Fig. 1B is a cross-section view simplified illustration of a diffractive surface of a multifocal IOL according to some embodiments of the present invention.

As shown in the exemplary embodiment depicted in Fig. 1A, An IOL 100 comprises a diffractive surface having a plurality of discrete, adjacent, diffractive, concentric sections or rings 102.

In some embodiments, the IOL diffractive surface comprises diffractive steps which are partially inside and partially outside the base curvature of the IOL. This induces changes in diffractive status between two steps. In some embodiments, the thickness of the IOL is variable but the curvature is maintained. In some embodiments, change in curvature is produced by optimization of the sphericity between steps.

Potential advantages in the described design are in that:
1. The designing of the diffractive steps does not change the diffractive profile in between the steps. This allows to have multifocal IOL of any added power and more efficiency for near focal lengths without losing contrast for distance.
2. Design of diffractive steps allows rays of light to be parallel to the step which is the ideal condition for diffraction.

In some embodiments, the rings are distributed along Fresnel zones (102-1/102-2). In some embodiments, and a shown in Fig. 1B, an IOL surface150 topography features repetitive triangular forms, squared forms or parabolic forms depending on the type of lens.

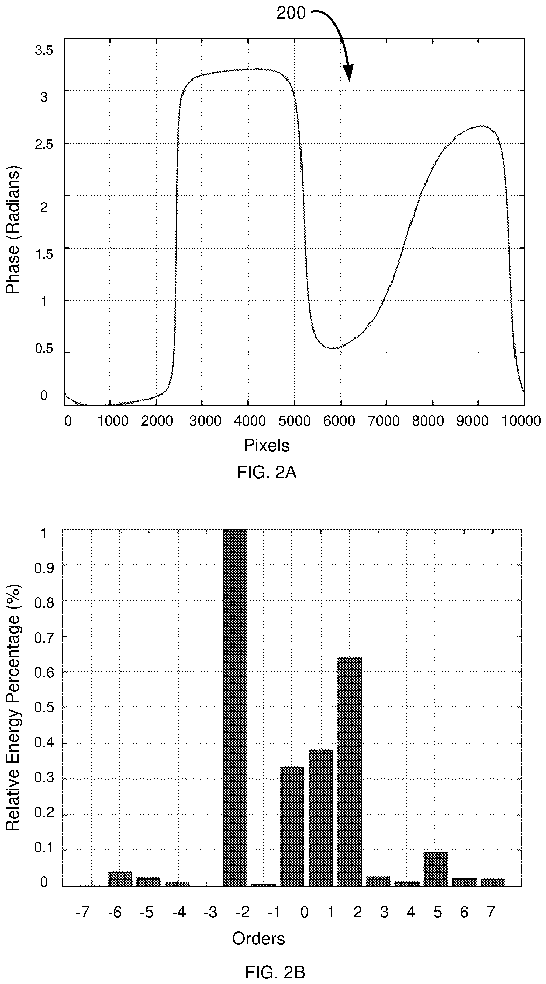

Reference is now made to Fig. 2A which is a radial, cross-section, simplified illustration of a diffractive surface topography of a portion of the IOL and Fig. 2B, which is a graph of distribution of energy flux (power) levels over specific diffractive orders associated with the diffractive surface topography shown in Fig. 2A.

Fig. 2A depicts a radial cross-section or radial phase profile 200 of a diffractive surface topography through a concentric ring. The specific cross-section of the radial phase profile is generated by using a Gerchberg-Saxton (GS) iterative algorithm. Fig. 2A depicts an exemplary embodiment of a radial phase profile cross-section showing an asymmetrical double-peaked cross-section having a near-symmetric local diffractive surface topography. Radial phase profile 200 is converted to a height profile which is radially and parabolically integrated to each of the Fresnel zones (rings) of the diffractive lens with a specific focal length.

The authors of this disclosure have come to learn that symmetric local diffractive surface topography function combined with an odd number of diffraction diffractive orders e.g., 1, 3, 5, 7 or 9 diffraction diffractive orders, and specifically a number of diffraction diffractive orders greater than three increase the overall light transmission efficiency of the IOL above 90% and in some cases up to at least 93%.

According to the claimed subject-matter, IOL 100 comprises five diffractive orders on the optical axis. According to the claimed subject-matter, the far vision focal length has the highest energy flux level. According to the claimed subject-matter, the diffractive order -1 is entirely suppressed. According to the claimed subject-matter, the five diffractive orders or foci correspond to the following five diffractive orders: -2, -1, +1 and +2 which are diffracted diffractive orders produced by the diffractive pattern and a single order 0 which is the refractive power produced by the spherical / aspherical surfaces of the IOL. According to the claimed subject-matter, the spherical / aspherical surfaces of the lens are designed such as to focus an image on the retina when the imaged object is located at an intermediate distance from the eye, between near vision (~30-40cm) and far vision (several meters).

According to the claimed subject-matter, and as depicted in Fig. 2B, of the five diffractive orders, the diffractive order -2, corresponds to far vision has the highest energy flux level. In some embodiments, of the remaining diffractive orders, the diffractive order +2, which corresponds to near vision has the highest energy flux level. According to the claimed subject-matter, the diffractive order -1 is suppressed.

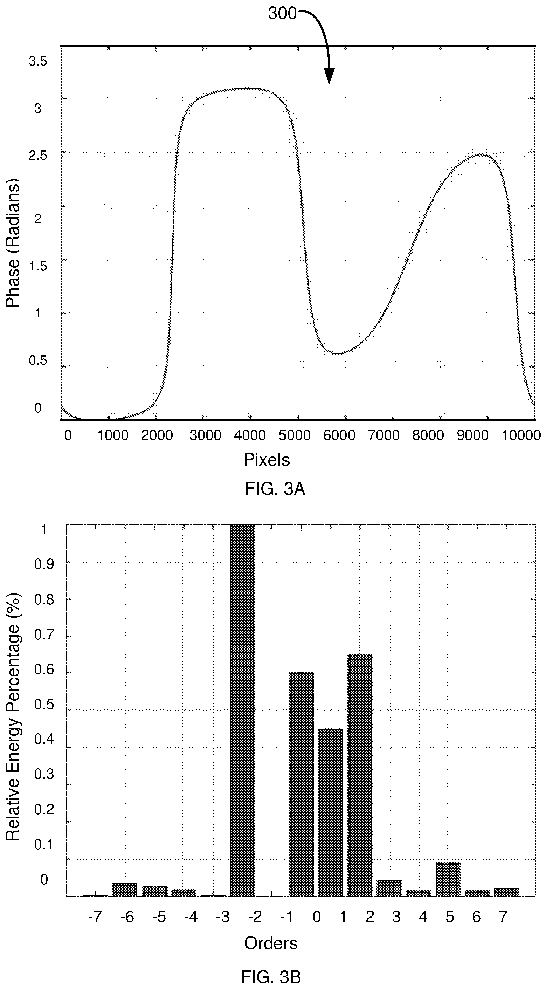

Reference is now made to Fig. 3A which is a radial, cross-section, simplified illustration of a diffractive surface topography of a portion of the IOL and Fig. 3B, which is a graph of energy flux levels distribution over specific diffractive orders associated with the diffractive surface topography shown in Fig. 3A.

Figures 3A and 3B show a design different than that illustrated in Figures 2A and 2B however both have been designed using the same method of profile generation by using a Gerchberg-Saxton (GS) iterative algorithm and conversion of the phase profile to a height profile which is radially and parabolically integrated to each of the Fresnel zones (rings) of the diffractive lens with a specific focal length.

Fig. 3A depicts an additional example of a radial cross-section or radial phase profile 300 of a diffractive surface topography through a concentric ring. Fig. 3A depicts an exemplary embodiment of a radial phase profile showing an asymmetrical double-peaked cross-section having a near-symmetric local diffractive surface topography. Radial phase profile 300 is converted to a height profile which is radially and parabolically integrated to each of the Fresnel zones (rings) of the diffractive lens with a specific focal length.

According to the claimed subject-matter, and as depicted in Fig. 3B, of the five diffractive orders, the diffractive order -2, corresponds to far vision has the highest energy flux level. In some embodiments, of the remaining diffractive orders, the diffractive order +2, which corresponds to near vision has the highest energy flux level and the energy flux level at order 0 is higher than the energy flux level at diffractive order +1. According to the claimed subject-matter, the diffractive order -1 is suppressed.

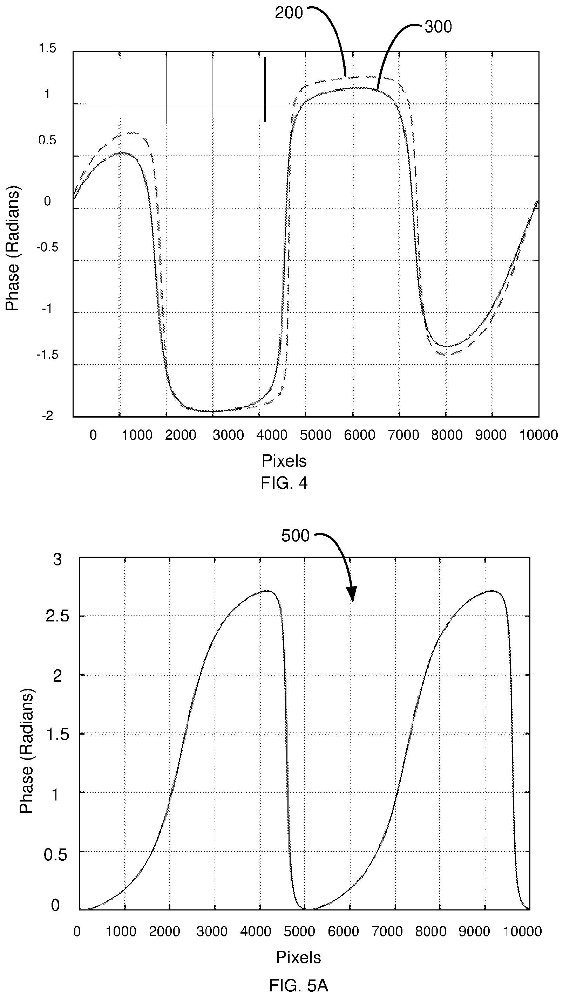

As shown in Fig. 4, which is a graph superimposing radial phase profile 300 over radial phase profile 200 demonstrates phase differences between the designs in accordance with some examples of the invention. When using this technique with more than 5 foci (for example 7), the diffractive focal length determines the location of the far focus (diffractive order -3) and near focus (diffractive order +3) and the refractive focal length (spherical surfaces) determines the location of one of the intermediate foci (order 0). In the exemplary embodiment depicted in Fig. 4, radial phase profile 200 comprises phases: 1, 0 ,0.333, 0.38 and 0.637, wherein radial phase profile 300 comprises phases: 1, 0, 0.6, 0.45 and 0.65.In some embodiments, the IOL comprises two zones - a central zone 102-1 (Figs. 1A and Fig. 1B) and a peripheral zone 102-2. In some embodiments, the diffractive pattern of central zone 102-1 is not apodized. In some environments, the diffractive pattern of the peripheral zone 102-2 is not apodized. The height of the diffractive surface topography of zone 102-2 is maintained constant and does not have a gradual step height reduction when advancing radially outwards in respect to the center height of the IOL. This arrangement and design are planned in diffractive order to vary the intensity distribution when increasing the aperture and allow increased light flux, even if not optimally focused, with pupil dilation (low intensity environmental) light.

A potential advantage in this design is in that the apodization design allows for diffraction efficiency near 100% thus minimizing loss of light.

For modifying the intensity distribution according to the aperture size, the technique used here is to change the diffractive pattern at any radius of the intraocular lens or at one of the Fresnel Zone (or near one of them) for better performance. For this invention, the design transition occurred at radius ~1.228mm.

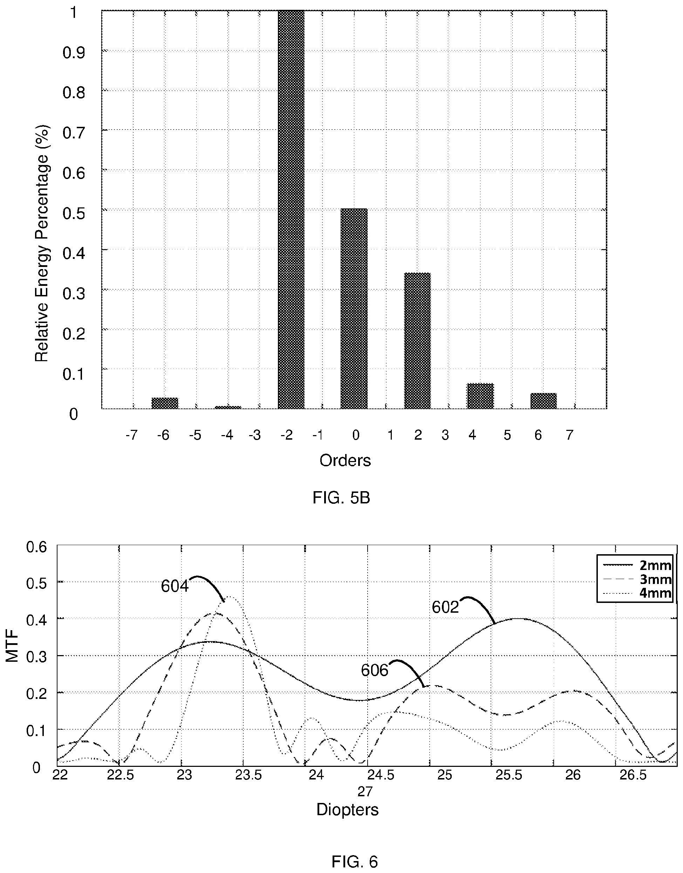

Fig. 5A and 5B correspond to exemplary embodiments not according to the claimed subject-matter. As shown in Figs. 5A and 5B, in which Fig. 5A depicts an example of a radial cross-section or radial phase profile 500 of a diffractive surface topography through a concentric ring of the peripheral zone of the IOL in accordance with some embodiments of the invention. Fig. 5A depicts an exemplary embodiment of a radial phase profile cross-section at the IOL peripheral zone 102-2 showing an asymmetrical single-peaked cross-section having a near-symmetric local diffractive surface topography.

In some embodiments, a radial phase profile cross-section at the IOL peripheral zone 102-2 comprises an asymmetrical double-peaked cross-section having a near-symmetric local diffractive surface topography. Radial phase profile 500 is converted to a height profile which is radially and parabolically integrated to each of the Fresnel zones (rings) of the diffractive lens with a specific focal length.

In some embodiments, and as depicted in Fig. 5B, the flux energy level declines from the far diffractive order to the near diffractive order with two suppressed orders at -1 and +1.

The phase obtained at the source plane and used in the design (at radius>1.228mm) depicted in Figs. 5A and 5B is obtained using normalized target intensity [1, 0, 0.5, 0, 0.34] in the Gerchberg-Saxton (GS) algorithm.

However, in some examples, the other diffractive profile (after transition) can be a different diffractive design based on three or more foci and can be also a monofocal design with full energy at far vision or bifocal with energy at far vision and other foci at intermediate or near vision

Also, for fine tuning of the intraocular lens, the entire diffractive height profile (before and after transition from IOL zone 102-1 to IOL zone 102-2) can be tunable. In the exemplary embodiment depicted in Figs. 5A and 5B, the first diffractive profile before the transition has been increased by 5% and the second diffractive profile after the transition has been increased by 12%. Our multifocal IOL, based on zero order for intermediate vision, has a high efficiency- over 90% - in the used diffractive orders (-2, -1, 0, 1, 2).

Reference is now made to Fig. 6, which is a graph of a simulation of a through-focus Modulation Transfer Function (MTF) of the IOL in accordance with some embodiments of the invention. As shown in the exemplary embodiment depicted in Fig. 5, three graphs are drawn for three levels of eye pupil diameter: graph 602 for a 2mm pupil diameter (i.e., intense light condition), graph 604 for a 4.5 mm pupil diameter (i.e., low light condition) and graph 606 for a 3 mm pupil diameter (i.e., normal light condition).

The graphs depicted in Fig. 6 exhibit the biphasic nature of the IOL at all three shown levels of environmental light however, as the level of environmental light decreases changing the pupil diameter from 2mm to 4.5mm, the MTF value correspondingly increases on the far-sighted range of the IOL, even though it is limited from a diopter range between approximately 22 and 24.5 to a narrower diopter range between approximately 23 to 23.75 and allows for an improved low-light intensity vision.

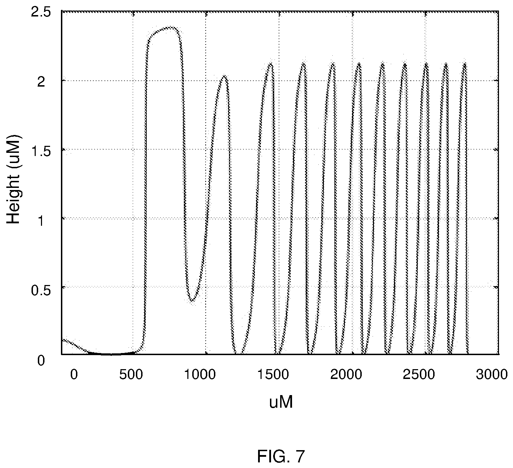

Fig. 7 which is a simplified graph illustration depicting a profile of a diffractive portion of an exemplary lens in accordance with some embodiments of the current invention. As shown in Fig. 7, the depicted cross-section profile comprises an asymmetrical single-peaked topography comprising a plurality of peaks having a height of between 1.5 and 2.5 uM dispersed along a portion of the lens radius between 0 and 3000 uM.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

In the description and claims of the application, each of the words "comprise" "include" and "have", and forms thereof, are not necessarily limited to members in a list with which the words may be associated.

The descriptions of the various embodiments of the present invention have been presented for purposes of illustration but are not intended to be exhaustive or limited to the embodiments disclosed. Many modifications and variations will be apparent to those of ordinary skill in the art without departing from the scope of the claims. The terminology used herein was chosen to best explain the principles of the embodiments, the practical application or technical improvement over technologies found in the marketplace, or to enable others of ordinary skill in the art to understand the embodiments disclosed herein.

## Claims

1. A multifocal intraocular lens, IOL, comprising:
at least one diffractive surface including a plurality of discrete, adjacent, diffractive, concentric Fresnel zones;
a diffractive profile producing five diffractive orders corresponding to -2, -1, 0, +1, +2; wherein the 0 order provides an intermediate vision and only the diffractive order -1 is suppressed among said five diffractive orders;
the diffractive order -2 has the highest energy flux level among the five diffractive orders; and
wherein light transmission efficiency of the IOL is above 90%.

2. The multifocal IOL according to claim 1, wherein of the remaining diffractive orders besides the diffractive order -2, the diffractive order +2 has the highest energy flux of said five diffractive orders.

3. The multifocal IOL according to claim 1, wherein of the remaining diffractive orders besides the diffractive order -2, the diffractive order +2 has the highest energy flux of said five diffractive orders and the energy flux level at order 0 is higher than the energy flux level at diffractive order +1.

4. The multifocal intraocular lens, IOL, according to claim 1, wherein said diffractive surface comprises diffractive steps; said steps are partially inside and partially outside the base curvature of the IOL.

5. The multifocal intraocular lens, IOL, according to claim 1, wherein said profiles of said Fresnel zones are asymmetrical.

6. The multifocal intraocular lens, IOL, according to any one of claims 1-5, wherein thickness of the IOL is variable and the curvature is maintained.

7. The multifocal intraocular lens, IOL, according to any one of claims 1-6, wherein thickness of the IOL is variable and the curvature is variable.

8. The multifocal intraocular lens, IOL, according to claim 1, wherein said efficiency is at least 93%.

## Patentansprüche

1. Multifokale Intraokularlinse, IOL, umfassend:
mindestens eine diffraktive Fläche, die eine Vielzahl von diskreten, benachbarten, diffraktiven, konzentrischen Fresnel-Zonen aufweist;
ein diffraktives Profil, das fünf Beugungsordnungen erzeugt, die den Ordnungen -2, -1, 0, +1, +2 entsprechen; wobei die Ordnung 0 ein Sehen im Intermediärsehen bereitstellt und von den genannten fünf Beugungsordnungen nur die Beugungsordnung -1 unterdrückt ist;
wobei die Beugungsordnung -2 den höchsten Energieflusspegel unter den fünf Beugungsordnungen aufweist; und wobei der Lichttransmissionswirkungsgrad der IOL über 90 % liegt.

2. Multifokale IOL nach Anspruch 1, wobei von den übrigen Beugungsordnungen außer der Beugungsordnung -2 die Beugungsordnung +2 den höchsten Energiefluss der genannten fünf Beugungsordnungen aufweist.

3. Multifokale IOL nach Anspruch 1, wobei von den übrigen Beugungsordnungen außer der Beugungsordnung -2 die Beugungsordnung +2 den höchsten Energiefluss der genannten fünf Beugungsordnungen aufweist und der Energieflusspegel bei der Ordnung 0 höher ist als der Energieflusspegel bei der Beugungsordnung +1.

4. Multifokale Intraokularlinse, IOL, nach Anspruch 1, wobei die genannte diffraktive Fläche diffraktive Stufen umfasst; wobei die genannten Stufen teilweise innerhalb und teilweise außerhalb der Grundkrümmung der IOL liegen.

5. Multifokale Intraokularlinse, IOL, nach Anspruch 1, wobei die genannten Profile der genannten Fresnel-Zonen asymmetrisch sind.

6. Multifokale Intraokularlinse, IOL, nach einem der Ansprüche 1 bis 5, wobei die Dicke der IOL veränderlich ist und die Krümmung beibehalten wird.

7. Multifokale Intraokularlinse, IOL, nach einem der Ansprüche 1 bis 6, wobei die Dicke der IOL veränderlich ist und die Krümmung veränderlich ist.

8. Multifokale Intraokularlinse, IOL, nach Anspruch 1, wobei der genannte Wirkungsgrad mindestens 93 % beträgt.

## Revendications

1. Lentille intraoculaire multifocale, LIO, comprenant :
au moins une surface diffractive comportant une pluralité de zones de Fresnel discrètes, adjacentes, diffractives et concentriques;
un profil diffractif produisant cinq ordres de diffraction correspondant à -2, -1, 0, +1, +2; dans laquelle l'ordre 0 procure une vision intermédiaire et, parmi lesdits cinq ordres de diffraction, seul l'ordre de diffraction -1 est supprimé;
dans laquelle l'ordre de diffraction -2 présente le niveau de flux énergétique le plus élevé parmi les cinq ordres de diffraction; et dans laquelle l'efficacité de transmission lumineuse de la LIO est supérieure à 90%.

2. LIO multifocale selon la revendication 1, dans laquelle, parmi les ordres de diffraction restants autres que l'ordre de diffraction -2, l'ordre de diffraction +2 présente le flux énergétique le plus élevé desdits cinq ordres de diffraction.

3. LIO multifocale selon la revendication 1, dans laquelle, parmi les ordres de diffraction restants autres que l'ordre de diffraction -2, l'ordre de diffraction +2 présente le flux énergétique le plus élevé desdits cinq ordres de diffraction et le niveau de flux énergétique à l'ordre 0 est supérieur au niveau de flux énergétique à l'ordre de diffraction +1.

4. Lentille intraoculaire multifocale, LIO, selon la revendication 1, dans laquelle ladite surface diffractive comprend des marches diffractives; lesdites marches étant situées en partie à l'intérieur et en partie à l'extérieur de la courbure de base de la LIO.

5. Lentille intraoculaire multifocale, LIO, selon la revendication 1, dans laquelle lesdits profils desdites zones de Fresnel sont asymétriques.

6. Lentille intraoculaire multifocale, LIO, selon l'une quelconque des revendications 1 à 5, dans laquelle l'épaisseur de la LIO est variable et la courbure est maintenue.

7. Lentille intraoculaire multifocale, LIO, selon l'une quelconque des revendications 1 à 6, dans laquelle l'épaisseur de la LIO est variable et la courbure est variable.

8. Lentille intraoculaire multifocale, LIO, selon la revendication 1, dans laquelle ladite efficacité est d'au moins 93%.
